# EUROPÄISCHE PATENTSCHRIFT

(11) **EP 4 103 729 B1**
(45) Veröffentlichungstag und Bekanntmachung des Hinweises auf die Patenterteilung: **18.10.2023**
(21) Anmeldenummer: 20725081.2
(22) Anmeldetag: 03.04.2020
(51) Int. Cl.: C12N 9/02, C12P 13/12

(54) **BIOKATALYSATOR ALS KERNKOMPONENTE EINES ENZYMKATALYSIERTEN REDOXSYSTEMS ZUR BIOKATALYTISCHEN REDUKTION VON CYSTIN**
BIOCATALYZER AS A CORE COMPONENT OF AN ENZYME CATALYZED REDOX SYSTEM FOR THE BIOCATALYTIC REDUCTION OF CYSTINE
BIOCATALYSATEUR COMME COMPOSANT PRINCIPAL D'UN SYSTÈME REDOX CATALYSÉ PAR DES ENZYMES POUR LA RÉDUCTION BIOCATALYTIQUE DE LA CYSTINE

(43) Veröffentlichungstag der Anmeldung: 21.12.2022
(73) Patentinhaber: Wacker Chemie AG, 81737 München (DE)
(72) Erfinder: BORNHÖVD, Carsten, 81825 München (DE); JACH, Guido, 53639 Königswinter (DE); TORRES MONROY, Ingrid, 40223 Düsseldorf (DE); WELTERS, Peter, 41334 Nettetal (DE)
(74) Vertreter: Belz, Ferdinand
(86) Internationale Anmeldenummer: PCT/EP2020/059654
(87) Internationale Veröffentlichungsnummer: WO 2021/197632

(56) Entgegenhaltungen:
- CN-A- 101 899 460
- US-A1- 2019 055 586
- B. WIELES ET AL: "Purification and Functional Analysis of the Mycobacterium leprae Thioredoxin/Thioredoxin Reductase Hybrid Protein", JOURNAL OF BIOLOGICAL CHEMISTRY, Bd. 270, Nr. 43, 27. Oktober 1995 (1995-10-27), Seiten 25604-25606, XP055762105, ISSN: 0021-9258, DOI: 10.1074/jbc.270.43.25604 in der Anmeldung erwähnt
- GRAHAM H. COOMBS ET AL: "The Amitochondriate Eukaryote Trichomonas vaginalis Contains a Divergent Thioredoxin-linked Peroxiredoxin Antioxidant System", JOURNAL OF BIOLOGICAL CHEMISTRY, Bd. 279, Nr. 7, 20. November 2003 (2003-11-20), Seiten 5249-5256, XP055762304, ISSN: 0021-9258, DOI: 10.1074/jbc.M304359200

## Beschreibung

Die Erfindung betrifft ein Enzym zur Reduktion von Cystin zu Cystein, dadurch gekennzeichnet, dass es sich beim Enzym um ein Fusionsprotein handelt, das die Proteinaktivitäten von Thioredoxin (Protein i) mit der KEGG-Datenbank Nummer EC 1.8.4.8 oder EC 1.8.4.10 und Thioredoxin-Reduktase (Protein ii) mit der KEGG-Datenbank Nummer EC 1.8.1.9 umfasst, wobei die Aktivität des Fusionsproteins mindestens 100% der Aktivität einer Mischung der gleichen, aber nicht fusionierten Einzelproteine i und ii beträgt. Des Weiteren betrifft die Erfindung ein Verfahren zur enzymatischen Reduktion von Cystin in Cystein in Gegenwart eines Kofaktors unter Verwendung dieses Fusionsproteins.

Cystein, abgekürzt Cys oder C, ist eine α-Aminosäure mit der Seitenkette -CH₂-SH. Da die natürlich vorkommende enantiomere Form L-Cystein ist und nur diese eine proteinogene Aminosäure darstellt, ist im Rahmen dieser Erfindung L-Cystein gemeint, wenn der Ausdruck Cystein ohne Deskriptor gebraucht wird. Durch Oxidation der Sulfhydrylgruppen können zwei Cysteinreste miteinander eine Disulfidbrücke bilden, womit Cystin entsteht, für das das gleiche gilt, d.h. ohne Deskriptor ist in dieser Erfindung das L-Enantiomer (oder R,R-Cystin) gemeint. L-Cystein ist für den Menschen eine semi-essenzielle Aminosäure, da sie aus der Aminosäure Methionin gebildet werden kann.

Die Aminosäure L-Cystein wird beispielsweise als Lebensmittelzusatzstoff (insbesondere in der Backmittelindustrie), als Einsatzstoff in der Kosmetik, sowie als Ausgangprodukt für die Herstellung von Pharmawirkstoffen (insbesondere N-Acetyl-Cystein und S-Carboxy-Methyl-Cystein) verwendet und ist damit von wirtschaftlicher Bedeutung. Als besonders wichtig wird der Einsatz von Cystein als Aromastoff in der Lebensmittelindustrie gesehen (z.B. als Hühnchen- oder Fleischaroma). Des Weiteren findet L-Cystein Anwendung zur Modifikation der Rheologie von Teigen und als Antioxidant z.B. in Säften.

In allen Organismen nimmt L-Cystein eine Schlüsselrolle im Schwefelmetabolismus ein. Es wird für die Synthese von Proteinen, Methionin, Biotin, Liponsäure, Gluthation und anderen schwefelhaltigen Metaboliten verwendet. Des Weiteren dient L-Cystein der Biosynthese von Coenzym-A. Die Thiolgruppe spielt in Enzymen häufig eine wichtige Rolle bei der Katalyse von Reaktionen oder stabilisiert Proteine durch die Ausbildung von intra- oder intermolekularen Disulfidbrücken.

L-Cystein kann mittels Hydrolyse aus keratinhaltigen Materialien wie Haaren, Borsten, Nägeln, Hufen, Federn und Hörnern gewonnen werden. Diese Verfahren bergen jedoch stets die Fragen nach biologischer Sicherheit und Umweltschutz. Zusätzlich weisen diese eine geringe Produktivität auf. Alternativ wurden Verfahren zur Herstellung mittels Biotransformation aus Vorstufen sowie die fermentative Herstellung von L-Cystein entwickelt (US 6,218,168 B1, US 5,972,663 A, US 2004-038352 A, CA 2 386 539 A2, EP 2 138 585, EP 1 769 080 und EP 2 726 625 B1). Nachteilig bei der fermentativen Produktion ist eine geringe Ausbeute von L-Cystein (auch nach optimierter Fermentationsführung), die auch darauf beruht, dass Cystein in Gegenwart von Luftsauerstoff oxidiert und dann hauptsächlich als Disulfid L-Cystin vorliegt bzw. gewonnen werden kann.

Die Oxidationsreaktion ist reversibel und so kann das L-Cystin durch eine gezielte Reduktion wieder in L-Cystein überführt werden. Wird jedoch L-Cystin nach Abtrennung von den Zellen (z.B. mit einem Dekanter) mittels Elektrolyse wieder zu L-Cystein reduziert, so bedingt diese chemische Umsetzung, dass solches L-Cystein nicht als natürlich nach Aromenverordnung deklariert werden darf.

Nach der EU-Aromenverordnung (1334/2008 Artikel 22 der Verordnung zur Neuordnung lebensmittelrechtlicher Kennzeichnungsvorschriften) sind natürliche Aromen wie folgt definiert: "Natürliche" Aromastoffe sind chemisch definierte Stoffe mit Aromaeigenschaften, die natürlich vorkommen und in der Natur nachgewiesen wurden. Sie werden durch geeignete physikalische, enzymatische oder mikrobiologische Verfahren aus pflanzlichen, tierischen oder mikrobiologischen Ausgangsstoffen gewonnen, die als solche verwendet oder mittels eines oder mehrerer herkömmlicher Lebensmittelzubereitungsverfahren für den menschlichen Verzehr aufbereitet werden.

In diesem Sinne wird der Begriff "natürlich" auch in vorliegender Anmeldung benutzt. Es besteht ein großes Interesse am Einsatz natürlicher Rohstoffe bei der Aromenherstellung.

Eine enzymatische Spaltung würde eine Herstellung eines *natürlichen* Cysteins unter Ausnutzung der hohen fermentativen Cystin-Ausbeuten ermöglichen. Für die Durchführung eines solchen enzymatischen Verfahrens bedarf es eines geeigneten Redox-Enzymkaskaden-Systems. Als Disulfid-spaltende Enzyme wurden z.B. Thioredoxine, Glutaredoxine und Disulfidisomerasen beschrieben. Meist wirken jedoch diese Enzyme auf Disulfide, die sich innerhalb eines Polypeptids oder zwischen zwei Untereinheiten eines Proteins ausgebildet haben. Die Spaltung von freiem Cystin ist nur für wenige Proteine beschrieben worden, wie z.B. für bakterielles Thioredoxin, dass wiederum durch die Thioredoxin-Reduktase, die NADPH als Kofaktor benötig, regeneriert wird. Solche Redox-Enzyme oder Enzymkaskaden benötigen häufig NADH oder NADPH als Kofaktor, der die entsprechenden Elektronen für die Reduktionsreaktion zur Verfügung stellt. Eine Cystin-Reduktase ist demnach dadurch charakterisiert, dass sie das Substrat Cystin verwendet und mit Hilfe eines Kofaktors wie NADPH oder NADH zwei Elektronen überträgt und schließlich zwei Cysteine entstehen.

Entsprechend muss der Kofaktor daher äquimolar der Reaktion zugesetzt werden, was kostenintensiv und damit wirtschaftlich ungünstig ist. Dies kann durch die Kopplung mit einer zweiten Reaktion zur Regeneration des Kofaktors vermieden werden. Als regeneratives System können Dehydrogenasen, wie z.B. Alkoholdehydrogenase oder Glukose-6-Phosphat-Dehydrogenase verwendet werden. Aus wirtschaftlicher Sicht kommt hinzu, dass auch diese Enzyme mit einer entsprechend hohen Ausbeute fermentativ herstellbar sein müssen.

Da der Aufwand zur Herstellung der benötigten Enzyme mit deren Anzahl steigt, ist es wirtschaftlich sinnvoll, die einzelnen Enzyme gentechnisch zu fusionieren. Dies kann zusätzlich den Vorteil haben, dass durch die räumliche Nähe die Substrate leichter von einem Enzym zum nächsten gelangen können. Häufig führen jedoch diese Enzymfusionen zu einer schlechteren fermentativen Produzierbarkeit oder das Fusionsprotein weist eine reduzierte oder sogar keine Aktivität mehr auf. Um eine inhibierende Wirkung der beiden Enzyme aufeinander im Fusionsprotein zu vermeiden werden häufig eher längere Linker-Sequenzen, die die beiden Proteine verbinden bzw. trennen, verwendet. Selten werden vergleichbare Aktivitäten der Fusionsproteine gegenüber Gemischen der Ausgangsproteine beobachtet, wenn sehr kurze Linker- oder gar keine Linkersequenzen verwendet CN101899460 offenbart mehrere Fusionsproteine, welche die Enzyme Thioredoxin (Trx) und Thioredoxin-Reduktase (TrxR) aus Aspergillus niger enthalten, wobei die beiden Enzyme durch einen "linker" von mehr als fünf Aminosäuren miteinander verbunden sind. Die Fusionsproteine werden in E.coli exprimiert, jedoch enthalten sie keine Aminosäuresequenzen, die aus E.coli stammen.

In *Mycobacterium leprae* (Wieles B. et al. 1995, J. Biol. Chem. 270, S. 25604-25606) findet sich ein Protein, das als Thioredoxin und Thioredoxin-Reduktase aktiv ist, wobei der N-Terminus des Proteins homolog zur Thioredoxin-Reduktase und der C-Terminus homolog zu Thioredoxin ist. Wenn man die beiden Proteinteile mit den homologen Proteinen aus *E.coli* vergleicht, sind diese in *M*. *leprae* über einen 22 Aminosäure-langen Spacer/Linker verbunden.

Aufgabe der vorliegenden Erfindung ist es, ein kostengünstig herstellbares Protein zur biokatalytischen Reduktion von Cystin in Cystein zur Verfügung zu stellen, dessen Aktivität mindestens genauso hoch ist, wie eine Mischung der im Stand der Technik bekannten Proteine Thioredoxin und Thioredoxin-Reduktase. Durch den Einsatz dieses Proteins in einem Verfahren zur enzymatischen Reduktion von Cystin in Cystein soll ein besonders effizientes und wirtschaftliches Verfahren bereitgestellt werden.

Diese Aufgabe wird gelöst durch die Bereitstellung eines Enzyms zur Reduktion von Cystin in Cystein, dadurch gekennzeichnet, dass es sich beim Enzym um ein Fusionsprotein handelt, das die Proteinaktivitäten von Thioredoxin (Protein i) mit der KEGG-Datenbank Nummer EC 1.8.4.8 oder EC 1.8.4.10, wobei es sich bei Thioredoxin (Protein i) um die Proteinaktivität von Thioredoxin 1 aus E.coli handelt, und Thioredoxin-Reduktase (Protein ii) mit der KEGG-Datenbank Nummer EC 1.8.1.9 umfasst, wobei es sich bei der Thioredoxin-Reduktase (Protein ii) um die Proteinaktivität der Thioredoxin-Reduktase aus E.coli handelt, wobei die Aktivität des Fusionsproteins mindestens 100% der Aktivität einer Mischung der gleichen, aber nicht fusionierten Einzelproteine i und ii beträgt, wobei das Fusionsprotein die Enzymaktivität aufweist, Cystin zu Cystein zu reduzieren und wobei die für die Aktivität von Protein i und ii verantwortlichen kodierenden Sequenzen (cds) fusioniert wurden.

Erfindungsgemäß handelt es sich beim Enzym zur Reduktion von Cystin in Cystein um ein Fusionsprotein, das die Proteinaktivitäten von Protein i und ii aus E.coli umfasst. Das bedeutet, dass die für die Aktivität von Protein i (Trx, beschrieben in der KEGG-Datenbank Nummer EC 1.8.4.8 oder EC 1.8.4.10) und die Aktivität von Protein ii (TR, beschrieben in der KEGG-Datenbank Nummer EC 1.8.1.9) verantwortlichen kodierenden Sequenzen (cds) fusioniert werden. Durch Entfernung des Stoppcodons hinter der ersten cds werden beide cds gemeinsam als eine cds exprimiert.

Die Proteinaktivität von Trx ist eine Oxidoreduktase, die eine Disulfidbrücke im aktiven Zentrum besitzt und in einem reduzierten oder oxidierten Zustand vorliegen kann.

Bei der Reaktion werden zwei Elektronen von den zwei Sulfhydrylgruppen auf eine Disulfidbrücke (intra- oder intermolekular) übertragen. Dabei wird eine intramolekulare Disulfidbrücke in Trx gebildet. Der reduzierte Zustand des Trx wird dann durch die katalytische Aktivität der Oxidoreduktase TR wiederhergestellt. Dazu sind Elektronen notwendig, die auf Trx unter Spaltung der Disulfidbrücke übertragen werden.

Ob ein Protein als Trx und/oder TR aktiv gegenüber Cystin ist (wenn das Protein beide Aktivitäten aufweist im Folgenden als Cystinreduktase/CR-Aktivität bezeichnet), kann mit Hilfe des folgenden Tests überprüft werden:
Zum einen kann photometrisch der Verbrauch an NADPH bei 340 nm gemessen werden. In einem Ansatz mit dem Fusionsprotein, dem Substrat Cystin und dem Kofaktor NADPH werden dann Elektronen von NADPH auf das Cystin übertragen und es wird Cystein gebildet. Der Verbrauch an NADPH kann dann photometrisch verfolgt werden.

Alternativ kann auch das entstehende Cystein in einem solchen Ansatz (aus Fusionsprotein, Cystin und NADPH) gemessen werden. Dabei wird ausgenutzt, dass die freie -SH Gruppe des Cysteins mit DTNB (5,5'-Dithiobis-2-nitrobenzoesäure; Ellman's Reagenz) reagiert und einen Farbstoff bildet. Diese farbige Verbindung Cys-TNB kann bei 412 nm gemessen werden. Mittels zeitlichen Verlaufs kann somit die Enzymaktivität des Fusionsproteins gegenüber Cystin gemessen werden.

Es handelt sich bei Thioredoxin (Protein i) um die Proteinaktivität von Thioredoxin 1 aus *E.coli.* (im Rahmen dieser Erfindung abgekürzt mit TrxA).

Es handelt sich bei der Thioredoxin-Reduktase (Protein ii) um die Proteinaktivität der Thioredoxin-Reduktase aus *E.coli* (im Rahmen dieser Erfindung abgekürzt mit TrxB).

Es ist insbesondere bevorzugt, dass das Fusionsprotein zwei Aminosäure-Sequenzen umfasst, wobei die eine Aminosäure-Sequenz eine Identität von mindestens 50%, bevorzugt mindestens 70% und besonders bevorzugt mindestens 90% zu SEQ ID Nr. 7 und die andere Aminosäure-Sequenz eine Identität von mindestens 50%, bevorzugt mindestens 70% und besonders bevorzugt mindestens 90% zu SEQ ID Nr. 8 hat und wobei das Fusionsprotein CR-Aktivität besitzt.

Entsprechend wird das Fusionsprotein mit TrxAB oder TrxBA bzw. TrxB5A bezeichnet, wobei beim Fusionsprotein TrxAB das Protein TrxA N-terminal von TrxB liegt. Dagegen liegt beim Fusionsprotein TrxBA bzw. TrxB5A das Protein TrxB N-terminal von TrxA. Es ist bevorzugt, dass es sich beim Fusionsprotein um TrxAB, TrxBA oder TrxB5A handelt. Es handelt sich beim Fusionsprotein um eine Fusion der Aminosäuresequenzen von Thioredoxin A und Thioredoxin B aus *E.coli,* bevorzugt um eine der Aminosäure-Sequenzen ausgewählt aus der Gruppe bestehend aus SEQ ID Nr. 9, SEQ ID Nr. 10 und SEQ ID Nr. 28.

In einer besonders bevorzugten Ausführungsform handelt es sich beim Fusionsprotein um TrxBA, insbesondere bevorzugt um die Aminosäure-Sequenz mit der SEQ ID Nr. 10.

Der Grad der DNA-Identität wird durch das Programm "nucleotide blast", zu finden auf der Seite http://blast.ncbi.nlm.nih.gov/, bestimmt, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind: Max target sequences = 100; Short queries = "Automatically adjust parameters for short input sequences"; Expect Treshold = 10; Word size = 28; Automatically adjust parameters for short input sequences = 0. Die entsprechenden voreingestellten Scoring Parameter sind: Match/Mismatch Scores = 1,-2; Gap Costs = Linear.

Für den Vergleich von Proteinsequenzen wird das Programm "protein blast", auf der Seite http://blast.ncbi.nlm.nih.gov/, genutzt. Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind: Max target sequences = 100; Short queries = "Automatically adjust parameters for short input sequences"; Expect Treshold = 10; Word size = 3; Automatically adjust parameters for short input sequences = 0. Die voreingestellten Scoring Parameter sind: Matrix = BLOSUM62; Gap Costs = Existence: 11 Extension: 1; Compositional adjustments = Conditional compositional score matrix adjustment.

Im Rahmen dieser Erfindung beginnen die Proteine wie TrxA, TrxB, TrxAB, TrxBA, TrxB5A oder Ml-TrxBA mit einem Großbuchstaben, während die diese Proteine kodierenden Sequenzen (auch mit cds abgekürzt) mit einem Kleinbuchstaben bezeichnet werden (trxA, trxB, trxAB, trxBA, trxB5A oder Ml-trxBA).

Der Begriff Fusionsprotein bedeutet im Rahmen der Erfindung, dass, das Protein die beiden Einzelproteine Thioredoxin (Trx) und Thioredoxin-Reduktase (TR) umfasst. Es wird von einem Gen kodiert, dessen kodierende Region die kodierende Region für Trx und die kodierende Region von TR umfasst, so dass diese gemeinsam als eine Einheit transkribiert und zu einem Polypeptid translatiert werden.

Ein besonderer Vorteil bei der Verwendung eines Fusionsproteins, das Trx und TR enthält, gegenüber der Verwendung der zwei einzelnen Proteine Trx und TR liegt in der räumlichen Lage der zwei Proteine: TR überträgt Elektronen auf Trx, wobei Trx und TR nicht erst in einem dreidimensionalen Diffusionsprozess aufeinander treffen müssen, sondern im Fusionsprotein mit einander verbunden sind. D.h. durch die räumliche Nähe können die Elektronen leichter von einem Enzym zum anderen gelangen. Während die Einzelproteine separat aufgereinigt werden müssen, werden die Enzymaktivitäten im Fusionsprotein gemeinsam isoliert, was ebenso ein großer wirtschaftlicher Vorteil ist.

Bevorzugt umfasst das Fusionspotein zusätzlich eine Sequenz als Hilfe zur Aufreinigung wie besonders bevorzugt ein Polyhistidin-Tag (His-tag). Ein His-tag ist ein Protein-Anhang, der zur Proteinreinigung und zum Nachweis markierter Proteine verwendet werden kann. Die Aminosäuresequenz des Polyhistidin-Tags ist eine Folge von mindestens sechs Histidinen (6HIS), deren Gensequenz N-terminal nach dem Start-Methionin-Codon oder C-terminal vor das Stopcodon in die cds kloniert wird. Dadurch entsteht ein Fusionsprotein mit einem Polyhistidin-Tag. Ein Fusionsprotein aus den Proteinen Trx und TR kann unter Klonierung nur eines His-tags aufgereinigt werden. Es sind also nicht nur weniger Reaktionsschritte für die Aufreinigung nötig, sondern auch die Klonierungsstrategie vereinfacht sich.

Außerdem ist es möglich, eine Schnittstelle für eine Protease oder ein Intein zwischen Polypeptidsequenz und His-tag einzufügen, um eine Abspaltung des His-tags nach einer Proteinreinigung zu ermöglichen.

Es ist bevorzugt, dass im Fusionsprotein die Aminosäuresequenz des N-terminal gelegenen Proteins C-terminal um eine bis maximal fünf Aminosäuren verkürzt ist. Es ist besonders bevorzugt, dass im Fusionsprotein die Aminosäuresequenz des N-terminal gelegenen Proteins C-terminal um eine Aminosäure verkürzt ist.

Es ist bevorzugt, dass im Fusionsprotein die Aminosäuresequenz des C-terminal gelegenen Proteins N-terminal um ein bis maximal fünf Aminosäuren, besonders bevorzugt um eine Aminosäure, verkürzt ist. Besonders bevorzugt fehlt dem im Fusionsprotein C-terminal gelegenen Protein das Startcodon. Letzteres hat den Vorteil, dass die Gefahr, dass an dieser Stelle neu mit der Translation begonnen wird, nicht gegeben ist.

Zudem ist es bevorzugt, dass im Fusionsprotein die Aminosäure-sequenzen der fusionierten Proteinaktivitäten von Thioredoxin (Protein i) und Thioredoxin-Reduktase (Protein ii) durch eine Linkersequenz von einer bis höchstens fünf Aminosäuren, besonders bevorzugt von einer Aminosäure verbunden ist.

Es ist insbesondere bevorzugt, dass im Fusionsprotein die Aminosäuresequenzen der fusionierten Proteine Thioredoxin (Protein i) und Thioredoxin-Reduktase (Protein ii) direkt aufeinander folgen, d.h. keine Linkersequenz vorhanden ist.

Wie oben bereits ausgeführt ist es von Vorteil, gemeinsam agierende Proteine in einem Fusionsprotein zusammenzubringen, um deren Klonierung, Aufreinigung und Aktivität zu verbessern. Um jedoch die Faltung bzw. die in diesem Fall nötige Interaktion der beiden Fusionsproteine nicht zu behindern bietet sich die Verwendung einer langen Linkersequenz an. Überraschenderweise wurde jedoch gefunden, dass in diesem Fall eine direkte Fusion der beiden Proteine (Trx, TR) zu einem funktionalen Enzympaar führte. Dies wird unterstrichen durch die Feststellung, dass die Aktivität des Fusionsproteins von der Reihenfolge der Fusion abhängt. So wies das Fusionsprotein TrxAB eine mit einer Mischung der Einzelproteine vergleichbare Aktivität auf.

Beim Fusionsprotein TrxAB (SEQ ID Nr. 9) fehlt in der TrxA-Sequenz (SEQ ID Nr. 7) das Stoppcodon, während von TrxB (SEQ ID Nr. 8) die erste Aminosäure (Methionin, M) deletiert wurde. Zudem trägt das Protein einen His-tag (Aminosäure 1-12 in SEQ ID Nr. 9). Zwischen der letzten Aminosäure von TrxA (Ala) und der nach Entfernen des Startcodons ersten Aminosäure von TrxB (Gly) ist keine Linkersequenz vorhanden.

Beim Fusionsprotein TrxBA (SEQ ID Nr. 10) fehlt in der TrxB-Sequenz (SEQ ID Nr. 8) die letzte Aminosäure (Lysin) und das Stoppcodon, während von TrxA (SEQ ID Nr. 7) die erste Aminosäure (Methionin, M) deletiert wurde. Zudem trägt das Protein einen His-tag (Aminosäure 1-12 in SEQ ID Nr. 10). Zwischen der vorletzten Aminosäure von TrxB (Ala) und der ersten Aminosäure von TrxA (Ser) ist eine Linkersequenz von einer Aminosäure (Gly) vorhanden.

Für das Fusionsprotein TrxB5A (SEQ ID Nr. 28) gilt das gleiche wie für TrxBA beschrieben, mit der Ausnahme, dass eine Linkersequenz von fünf Aminosäuren (Gly-Pro-Ala-Pro-Gly) zwischen TrxB und TrxA vorhanden ist.

Der für die Aminosäuresequenz des Fusionsproteins kodierende Bereich, der zwischen einem Start- und einem Stoppcodon liegt, wird als kodierende Sequenz (coding sequence, cds) bezeichnet. Cds werden von nicht-kodierenden Bereichen umgeben. Als Gen wird der DNS-Abschnitt bezeichnet, der alle Grundinformationen zur Herstellung eines biologisch funktionellen Proteins enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle. Des Weiteren ist eine Expressionsregulation durch einen Terminator und ein oder mehrere Operatoren möglich.

Für einen funktionellen Promotor gilt, dass die unter der Regulation dieses Promotors stehende cds in eine RNS transkribiert wird.

Der DNS-Abschnitt kodierend Protein i des Fusionsproteins (Thioredoxin) kann zunächst mittels PCR unter Verwendung von Oligonukleotiden als Primer und einer DNS-Matrize, kodierend Thioredoxin, beispielsweise genomische DNS isoliert aus *E.coli,* amplifiziert und anschließend mit gängigen molekularbiologischen Techniken jeweils mit dem DNS-Molekül, das die Sequenz von Protein ii des Fusionsproteins (Thioredoxin-Reduktase) umfasst und das auf analoge Weise erzeugt wurde, derart verknüpft werden, dass eine *in* frame-Fusion entsteht. Das im Fusionsprotein zwischen den Proteinen i und ii vorhandene zweite Startkodon des C-terminal gelegenen Fusionspartners kann deletiert werden, um einen alternativen Leserahmen zu vermeiden. An der Übergangsstelle der beiden Fusionspartner können unterschiedlich lange Linkersequenzen eingefügt werden.

Alternativ zur Fusion über Klonierungsstellen kann das gesamte DNS-Molekül mittels Gensynthese hergestellt werden. Dieses DNS-Molekül kann dann entweder in einen Vektor, z.B. ein Plasmid, eingebracht werden oder direkt nach bekannten Methoden in das Chromosom des Bakterienstamms integriert werden. Bevorzugt wird das DNS-Molekül in ein Plasmid eingebracht, wie etwa einem Abkömmling von bekannten Expressionsvektoren wie pJF118EH, pKK223-3, pUC18, pBR322, pACYC184, pASK-IBA3, pGJ3477 oder pET.

Es ist bevorzugt, dass das DNS-Molekül hauptsächlich die kodierende Sequenz des Fusionsproteins und wenige weitere Nukleotide trägt und so in das Plasmid kloniert wird, dass dieses in einem bestimmten Abstand zum Promotor und Terminator kloniert wird, d.h. das DNS-Molekül wird 3` einer den Promotor kodierenden Region und 5` einer Terminator kodierenden Region in das Plasmid kloniert.

Als Promotoren eignen sich alle dem Fachmann bekannten Promotoren, wie konstitutive Promotoren, z.B. der GAPDH-Promotor, oder induzierbare Promotoren wie z.B. der lac-, tac-, trc-, lambda PL, araB-, cumate- oder tet-Promotor oder davon abgeleitete Sequenzen. Besonders bevorzugt wird das Fusionsprotein unter Kontrolle des durch Arabinose induzierbaren araB Promotors (P_{BAD}) exprimiert.

Die eingesetzten Plasmide können Selektionsmarker tragen. Als Selektionsmarker geeignet sind Gene, die für eine Resistenz gegenüber Antibiotika wie z.B. Ampicillin, Tetracyclin, Chloramphenicol, Kanamycin oder andere kodieren. Bevorzugt enthält das Plasmid ein Gen, dessen Expression Ampicillin-Resistenz vermittelt. Weiterhin sind Auxotrophie-Marker als Selektionsmarker geeignet, da diese für ein essentielles Gen im Stoffwechsel kodieren, das im jeweiligen Bakterienstamm, der das Plasmid enthält, deletiert ist.

Plasmide können unter Anwendung von dem Fachmann bekannten Methoden in die Zellen eines Bakterienstamms eingebracht werden (Transformation). Der Bakterienstamm ist bevorzugt dadurch gekennzeichnet, dass es sich bei dem Bakterienstamm um Gramnegative Bakterien, besonders bevorzugt um einen Bakterienstamm der Gattung Enterobacteriaceae, insbesondere bevorzugt um einen Stamm der Art *Escherichia coli* (*E.coli*) handelt.

Der das Plasmid enthaltene Bakterienstamm kann in einem Fermentationsverfahren verwendet werden, d.h. die Bakterienzellen können in Medium, bevorzugt in selektivem LB-Medium (entsprechend dem auf dem Plasmid enthaltenden Selektionsmarker) vermehrt, nach der Kultur durch Sedimentation und Verwerfen des Überstands vom Medium abgetrennt und lysiert werden. Das exprimierte Fusionsprotein kann dann beispielsweise mittels Affinitätschromatographie (z.B. über ein His-tag, s.o.) isoliert werden.

Das erfindungsgemäße Enzym hat den Vorteil, dass es fermentativ in hoher Ausbeute und daher kostengünstig herstellbar ist. Besonders wirtschaftlich effektiv und vorteilhaft ist die Herstellung auch deshalb, weil nur ein Fusionsprotein anstelle von zwei separaten Proteinen produziert, aufgereinigt und isoliert werden muss.

Es ist vorteilhaft, dass das Enzym durch einen Kofaktor wie NADPH oder NADH als Elektronendonor und damit als Reduktionsmittel regeneriert werden kann, da NADPH bzw. NADH regenerierende Systeme bereits existieren.

Die Enzymaktivitäten von Trx und TR können über den Umsatz des Kofaktors beispielsweise photometrisch bei 340 nm bestimmt werden. Dies ist möglich, da sich der Extinktionskoeffizient bei 340 nm beim Übergang vom reduzierten (NADPH/NADH) zum oxidierten Zustand (NADP/NAD) signifikant ändert.

Bei der Messung der Enzymaktivitäten der unterschiedlichen Konstrukte (Fusionsproteine) bzw. unterschiedlicher Mischungsverhältnisse der Einzelproteine war es erstaunlich und für den Fachmann nicht vorhersehbar, dass die Aktivität des Fusionsproteins mindestens 100%, bevorzugt mindestens 150% und insbesondere bevorzugt mindestens 200% der Aktivität einer Mischung der gleichen, aber nicht fusionierten Einzelproteine Thioredoxin und Thioredoxin-Reduktase beträgt. Das bedeutet, dass die Aktivität des Fusionsproteins genauso hoch oder höher ist, verglichen mit der Aktivität einer Mischung der gleichen, aber nicht fusionierten Einzelproteine i und ii.

Im Detail bedeutet dies, dass jeweils, außer den Negativkontrollen ohne Kofaktor oder Substrat, der gleiche Reaktionsansatz aus Substrat, Kofaktor und Puffersystem vorgelegt wurde. Nach Zugabe der gleichen Menge Fusionsprotein in einem Ansatz bzw. der Einzelproteine i (Trx) und ii (TR) in einem anderen Ansatz wurde der Kofaktor-Umsatz, in der gleichen Zeit unter Verwendung gleicher Reaktionsbedingungen bestimmt. Alternativ ist es genauso möglich, die sinkende Substratmenge oder steigende Produktmenge nachzuweisen.

Bevorzugt ist das Enzym dadurch gekennzeichnet, dass das Gen des Fusionsproteins eine DNS-Sequenz umfasst, von der eine Identität von mindestens 50%, bevorzugt mindestens 70% und besonders bevorzugt mindestens 90% zu SEQ ID Nr. 2 und die andere eine Identität von mindestens 50%, bevorzugt mindestens 70% und besonders bevorzugt mindestens 90% zu SEQ ID Nr. 3 besitzt.

In einer besonders bevorzugten Ausführungsform handelt es sich bei der das Fusionsprotein kodierenden DNS-Sequenz um SEQ ID Nr. 5.

Ein weiterer Gegenstand der Erfindung ist ein Verfahren zur enzymatischen Reduktion von Cystin in Cystein, dadurch gekennzeichnet, dass Cystein mit dem erfindungsgemäßen Enzym in Gegenwart eines Kofaktors reduziert wird.

Bei der enzymatischen Reduktion von Cystin in Cystein wird ein Disulfid (-S-S-) in zwei Sulfhydrylgruppen (-SH) umgewandelt, d.h. aus einem Molekül der chemischen Verbindung Cystin entstehen zwei Moleküle L-Cystein. Das erfindungsgemäße Fusionsprotein katalysiert die Reduktion und überträgt Elektronen auf das Disulfid des Cystins.

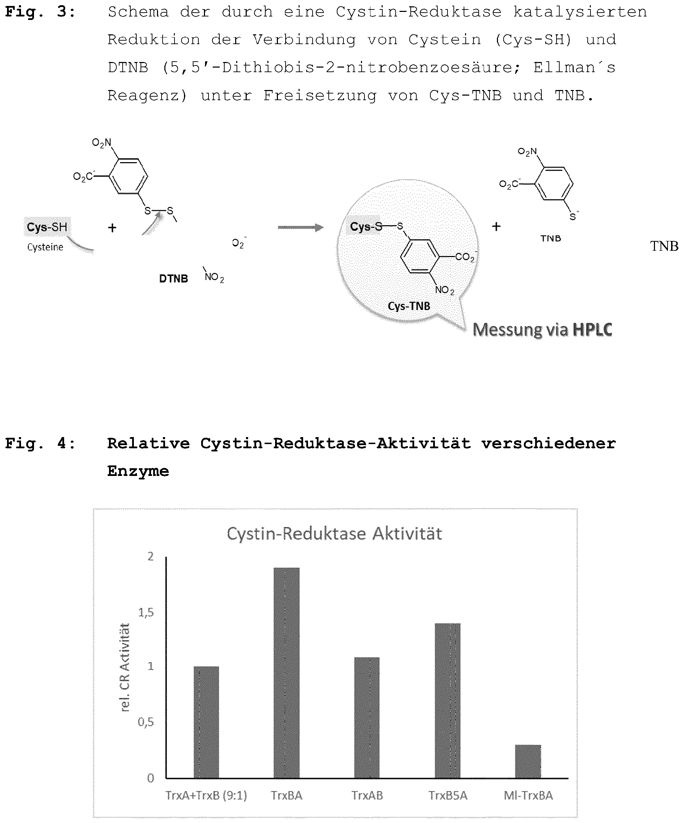

Dabei war es überraschend, dass insbesondere die Aktivität des Fusionsproteins TrxBA deutlich höher war, als die Aktivität einer Mischung der gleichen, aber nicht fusionierten Einzelproteine TrxA und TrxB (s. Beispiel 4, Fig. 4).

Ein besonderer Vorteil des erfindungsgemäßen Verfahrens liegt darin, dass das mit dem Verfahren hergestellte Cystein als natürliches Cystein gemäß Aromenverordnung deklariert werden kann.

Eine weitere Herausforderung für ein wirtschaftliches Verfahren bestand in der schlechten Löslichkeit des Cystins in Puffersystemen in einem neutralen pH-Bereich (bzw. in physiologischem pH-Bereich, d.h. bei einem pH-Wert von ~ pH 7,4).

Das Verfahren findet bevorzugt in einem pH-Bereich von 4 bis 11, besonders bevorzugt von 5 bis 10 und insbesondere bevorzugt von 6 bis 9 statt.

Es ist von besonderem Vorteil und daher darüber hinaus besonders bevorzugt, wenn das erfindungsgemäße Enzym in einem Verfahren zur enzymatischen Reduktion von Cystin in Cystein bei physiologischem pH-Wert eingesetzt wird.

Das Verfahren findet bevorzugt bei einer Temperatur von 20 bis 40 °C, besonders bevorzugt von 25 bis 30 °C statt.

Das Verfahren ist bevorzugt dadurch gekennzeichnet, dass es sich beim Kofaktor um eine Substanz ausgewählt aus der Gruppe bestehend aus NADPH und NADH handelt. Besonders bevorzugt handelt es sich beim Kofaktor um NADPH.

Bevorzugt ist das Verfahren dadurch gekennzeichnet, dass nach der Reduktion das gebildete Cystein isoliert wird. Zur weiteren Aufreinigung des Zielproduktes können folgende Schritte/Verfahren genutzt werden:
- Isolierung des L-Cysteins durch Ionentauscheradsorption
- Fällungskristallisation

Derartige Verfahren sind aus dem Stand der Technik bekannt (s. z.B. WO 2013/000864).

Bei der Reduktion von Cystin in Cystein wird das erfindungsgemäße Enzym oxidiert, d.h. es überträgt Elektronen an Cystin unter Ausbildung einer Disulfidbrücke. Da die Elektronen des anwesenden Kofaktors NADPH oder NADH durch die Aktivität des Fusionsproteins (TR-Anteil) auf das Fusionsprotein (Trx-Anteil) übertragen werden, entsteht NADP⁺ bzw. NAD⁺. Aus diesem Grund ist es bevorzugt, dass das Verfahren ein Kofaktor-regenerierendes Enzym enthält. Besonders bevorzugt handelt es sich beim Kofaktor-regenerierenden Enzym um eine Dehydrogenase, wobei die Reduktion zusätzlich in Gegenwart eines weiteren Elektronendonors erfolgt.

Beim Kofaktor-regenerierenden Enzym kann es sich um eine Glukose-6-Phosphat-Dehydrogenase und/oder die Alkoholdehydrogenase handeln. In einer besonders bevorzugten Ausführungsform handelt es sich bei der Dehydrogenase um die Alkoholdehydrogenase, wobei als Elektronendonor Isopropanol verwendet wird.

Die Erfindung wird im Folgenden anhand von Ausführungsbeispielen näher beschrieben.

### Beispiele

### Beispiel 1: Generierung des Cystinreduktase-Systems TrxA, TrxB, TrxBA und TrxAB

### Vorbereitung des Expressionsvektors:

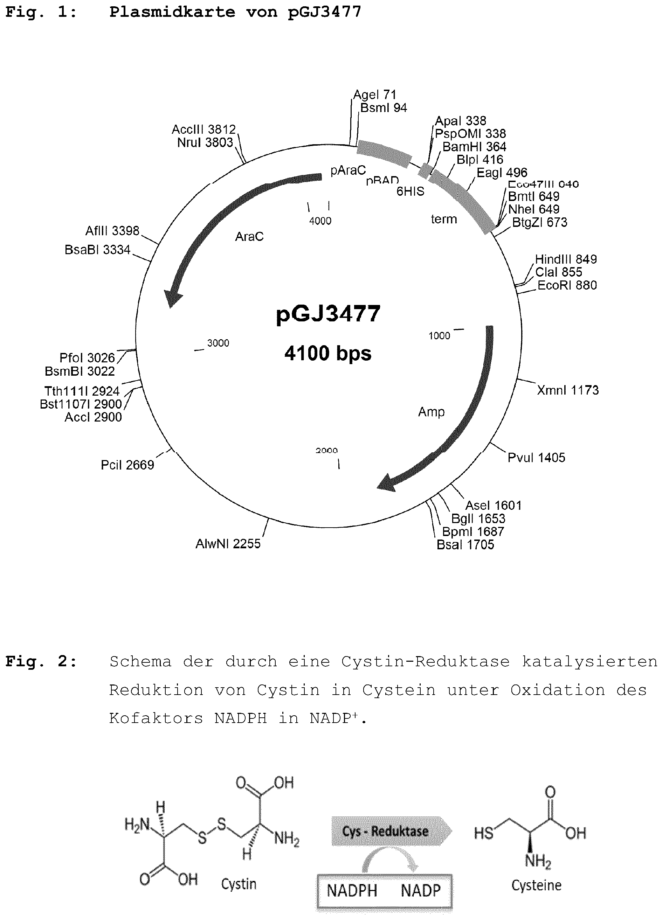

Als Vektor für die Expression der für die entsprechenden Kandidatenproteine TrxA, TrxB, TrxAB, TrxBA, TrxB5A oder Ml-TrxBA kodierenden DNS-Sequenzen wurde das Expressionsplasmid pGJ3477 ausgewählt. Dabei handelt es sich um ein Medium- bis high-copy Plasmid (50-60 Kopien pro Zelle) auf Basis des ColE1-Replika-tionsursprungs. Die Plasmidkarte ist in Fig. 1 dargestellt, wobei die Positionen gebräuchlicher, nur einmal schneidender Restriktionsenzyme (mit 6 Basen Erkennungssequenz) auf der Plasmidkarte angegeben sind. Die Sequenz ist in SEQ ID Nr. 11 angegeben.

In diesem Plasmid wurde die kodierende Sequenz des jeweiligen Kandidaten unter die Kontrolle des durch Arabinose induzierbaren Promotors P_{BAD} gestellt.

Zunächst wurde das gesamte Expressionsplasmid mittels Inverse PCR amplifiziert:
- 50 ng pGJ3477-DNS, 0,5 pmol der jeweiligen Primer 3477-fwrd (SEQ ID Nr. 12) und 3477-rev (SEQ ID Nr. 13), Q5^{®}-Reaktions-puffer (New England Biolabs, NEB), 1 Unit Q5^{®}-DNA-Polymerase (NEB) in einem Endvolumen von 50 ul.
- PCR-Programm: 1 min 98°C, dann 30 Zyklen mit 30 s 98 °C, 30 s 65 °C (Annealing) und 2 min 72°C (Synthese).

Nach Abschluss der Reaktion wurde dem Reaktionsmix das Restriktionsenzym DpnI (10 Units, NEB) zugesetzt und der Ansatz für 1 Stunde bei 37 °C inkubiert. Im Anschluss erfolgte die chromatographische Aufreinigung der DNS (Macherey & Nagel: NucleoSpin^{®} Gel und PCR Clean-up-Kit).

### Klonierung von trxA und trxB:

Zur Klonierung der Protein-kodierenden Sequenzen trxA (SEQ ID Nr. 2) und trxB (SEQ ID Nr. 3) wurden Oligonukleotidprimer bestimmt, deren Zielgenspezifische Sequenzen um mindestens 15 Nukleotide verlängert wurden, die überlappend mit den Sequenzen am Ende der Vector-DNS waren. Die Gene wurden mittels PCR aus dem *E.coli-*Genom von BL21 amplifiziert (Kolonie-PCR).

Zur Amplifikation mittels PCR wurden folgende Ansätze gewählt:
- 50 ng der genomischen *E.coli* BL21-DNS (NEB), 0,5 pmol der jeweiligen Primer trxA-fwrd (SEQ ID Nr. 14) und trxA-rev (SEQ ID Nr. 15) bzw. trxB-fwrd (SEQ ID Nr. 16) und trxB-rev (SEQ ID Nr. 17), Q5^{®}-Reaktionspuffer (NEB), 1 Unit Q5^{®}-DNA-Polymerase (NEB) in einem Endvolumen von 50 µl.
- PCR-Programm: 1 min 98°C, dann 30 Zyklen mit 30 s 98 °C, 30 s 60 °C (Annealing) und 15 s (trxA) oder 30 s (trxB) 72 °C (Synthese).

Nach Abschluss der Reaktion wurde dem Reaktionsmix das Restriktionsenzym DpnI (10 Units, NEB) zugesetzt und der Ansatz für 1 Stunde bei 37 °C inkubiert. Im Anschluss erfolgte die chromatographische Aufreinigung der DNS (Macherey & Nagel: NucleoSpin^{®} Gel und PCR Clean-up-Kit).

### LIC-PCR:

Generell wurden die Protein-kodierenden DNS-Sequenzen über LIC-PCR (ligation-independent-cloning of PCR products) wie beschrieben in Aslanidis C. und de Jong P. J., Nucleic Acids Res. 18, S. 6069-6074) Basen-genau in den Basis-Expressionsvektor pGJ3477 eingeführt.

Zu diesem Zweck wurden 50 ng der aufgereinigten kodierenden DNS des jeweiligen Kandidatenproteins zusammen mit 50 ng der vorbereiteten Vektor-DNS in die LIC-PCR-Reaktion eingesetzt.

Der LIC-PCR-Ansatz wurde dann mit Standardmethoden in *E.coli* XL1-blue-Zellen transformiert und auf selektivem LB-Medium ausplattiert (LB + 100 mg/L Ampicillin) und für 18 h bei 37 °C inkubiert. Zur Identifikation korrekter Klone wurde Plasmid-DNS aus den erhaltenen Kolonien isoliert und die Expressionskassette vollständig sequenziert.

Die resultierenden Plasmide, die aus dem Expressionsvektor pGJ3477 und der codierenden DNS-Sequenz trxA bzw. trxB zusammengesetzt waren, wurden im Folgenden als trxA- bzw. trxB-Expressionvektor bezeichnet.

### Klonierung von trxAB und trxBA:

Die Klonierung der das entsprechende Fusionsprotein TrxAB bzw. TrxBA kodierenden DNS trxAB und trxBA erfolgte analog der Klonierung von trxA und trxB wie oben beschrieben durch Insertion der trxA Sequenz in den trxB-Expressionsvektor mittels LIC-PCR zwischen den N-terminalen his-tag und die das Protein TrxB kodierende Sequenz trxB bzw. durch Insertion der trxB Sequenz in den trxA-Expressionvektor mittels LIC-PCR zwischen den his-tag und die das Protein TrxA kodierende Sequenz trxA:
Zur Amplifikation mittels PCR wurden folgende Ansätze gewählt:
- Die Vektor-DNS (trxA- bzw. trxB-Expressionvektor) wurde mittels Inverse PCR mit den Primern vtrxA-fwrd (SEQ ID Nr. 18) und 3477-rev (SEQ ID Nr. 13) bzw. vtrxB-fwrd (SEQ ID Nr. 21) and 3477-rev (SEQ ID Nr. 13) amplifiziert, wobei das folgende PCR-Programm gewählt wurde: 2 min 98 °C, dann 30 Zyklen mit 45 s 98 °C / 30 s 60 °C / 2,5 min 72 °C.
- Die trxA bzw. trxB Genabschnitte für die Fusionsproteine wurden mittels PCR mit den Primern ftrxA-fwrd (SEQ ID Nr. 22) und ftrxA-rev (SEQ ID Nr. 23) bzw. ftrxB-fwrd (SEQ ID Nr. 19) und ftrxB-rev (SEQ ID Nr. 20) amplifiziert, wobei das folgende PCR-Programm gewählt wurde: 2 min 98 °C -> dann 30 Zyklen mit 45 s 98 °C / 30 s 60 °C / 15 s 72 °C. Als Template wurde genomische DNS von BL21 verwendet.

Nach Abschluss der PCR-Reaktionen wurde dem jeweiligen Reaktionsmix das Restriktionsenzym DpnI (10 Units, NEB) zugesetzt und der Ansatz für 1 Stunde bei 37 °C inkubiert. Im Anschluss erfolgte die chromatographische Aufreinigung der Insert-DNS (Macherey & Nagel; NucleoSpin^{®} Gel and PCR Clean-up-Kit).

Für die LIC-PCR-Reaktion wurden 50 ng der amplifizierten Vektor-DNS des trxA- bzw. trxB-Expressionsvektors und 75 ng der Insert-DNS (trxB bzw. trxA) verwendet. Der LIC-PCR-Ansatz wurde dann mit Standardmethoden in *E.coli* XL1-blue-Zellen transformiert und auf selektivem LB-Medium ausplattiert (LB + 100 mg/L Ampicillin) und für 18 h bei 37 °C inkubiert. Zur Identifikation korrekter Klone wurde Plasmid-DNS aus den erhaltenen Kolonien isoliert und die Expressionskassette vollständig sequenziert.

SEQ ID Nr. 9 bzw. SEQ ID Nr. 10 geben die aus der Expression der Sequenzen SEQ ID Nr. 4 bzw. SEQ ID Nr. 5 resultierenden Aminosäuresequenzen der Fusionsproteine TrxBA und TrxAB an.

### Beispiel 2: Klonierung der Cystinreduktase Ml-TrxBA

Wie eingangs beschrieben besitzt *Mycobacterium leprae* einen Genabschnitt, der für das Protein mit Homologie zu Thioredoxin (Trx) und Thioredoxin-Reduktase (TR) kodiert (Wieles B. et al. 1995, J. Biol. Chem. 270, S. 25604-25606). Die Sequenz kann den öffentlichen Datenbanken entnommen werden (NCBI Reference Sequence: WP_010909042.1).

Die aus den öffentlichen Datenbanken entnommene Sequenz wurde *in-silico* auf die Kodon-Nutzung des Wirts *E.coli* angepasst. Hierfür wurde der Web-Server der Firma IDT eingesetzt (www.idtdna.com). Zur Klonierung in den Zielvektor pGJ3477 wurde die kodierende Region am 5'und 3'-Ende um Sequenzen verlängert, die mit der Vektorsequenz überlappen (siehe auch Klonierung trxA und trxB). Die resultierende DNS-Gesamtsequenz wurde von der Firma Geneart (www.thermofisher.com) synthetisch hergestellt (angegeben in SEQ ID Nr. 24) und mit Ml-trxBA (synonym ml-trxBA) bezeichnet.

Die Klonierung der synthetischen Ml-trxBA-Sequenz erfolgte in den Vektor pGJ3477 in Analogie zur Klonierung der trxA und trxB Expressionsvektoren mittels LIC-PCR.

Zur Klonierung der Protein-kodierenden Sequenz Ml-trxBA (SEQ ID Nr. 24) wurden Oligonukleotidprimer bestimmt, deren Zielgenspezifische Sequenzen um mindestens 15 Nukleotide verlängert wurden, die überlappend mit den Sequenzen am Ende der Vector-DNS waren. Als Template diente das synthetische Gen.

Zur Amplifikation mittels PCR wurden folgende Ansätze gewählt:
- 20 ng des Templates (Geneart), 0,5 pmol der jeweiligen Primer Ml-trxBA-fwrd (SEQ ID Nr. 25) und Ml-trxBA-rev (SEQ ID Nr. 26), Q5^{®}-Reaktionspuffer (NEB), 1 Unit Q5^{®}-DNA-Polymerase (NEB) in einem Endvolumen von 50 µl.
- PCR-Programm: 1 min 98°C, dann 30 Zyklen mit 30 s 98 °C, 30 s 60 °C (Annealing) und 60 s 72 °C (Synthese).

Nach Abschluss der Reaktion wurde dem Reaktionsmix das Restriktionsenzym DpnI (10 Units, NEB) zugesetzt und der Ansatz für 1 Stunde bei 37 °C inkubiert. Im Anschluss erfolgte die chromatographische Aufreinigung der DNS (Macherey & Nagel: NucleoSpin^{®} Gel und PCR Clean-up-Kit).

### LIC-PCR:

Die Protein-kodierende DNS-Sequenz wurde über LIC-PCR (ligation-independent-cloning of PCR products) wie beschrieben in Aslanidis C. und de Jong P. J., Nucleic Acids Res. 18, S. 6069-6074) Basen-genau in den Basis-Expressionsvektor pGJ3477 eingeführt.

Zu diesem Zweck wurden 50 ng der aufgereinigten kodierenden DNS zusammen mit 60 ng der vorbereiteten Vektor-DNS in die LIC-PCR-Reaktion eingesetzt.

Der LIC-PCR-Ansatz wurde dann mit Standardmethoden in *E.coli* XL1-blue-Zellen transformiert und auf selektivem LB-Medium ausplattiert (LB + 100 mg/L Ampicillin) und für 18 h bei 37 °C inkubiert. Zur Identifikation korrekter Klone wurde Plasmid-DNS aus den erhaltenen Kolonien isoliert und die Expressionskassette vollständig sequenziert.

Das resultierende Plasmid, das aus dem Expressionsvektor pGJ3477 und der codierenden DNS-Sequenz Ml-trxBA zusammengesetzt war, wurde im Folgenden als Ml-trxBA-Expressionvektor bezeichnet.

### Beispiel 3: Klonierung der Cystinreduktase TrxB5A (mit Linkersequenz)

### Klonierung von trxB5A:

Die Klonierung der das entsprechende Fusionsprotein TrxB5A kodierenden DNS trxB5A erfolgte analog der Klonierung von trxBA wie oben beschrieben durch Insertion einer um 5 Aminosäuren C-terminal verlängerten TrxB Sequenz (im Folgenden als TrxB5, bzw. die cds als trxB5 bezeichnet) in den trxA-Expressionsvektor mittels LIC-PCR zwischen den his-tag und die das Protein TrxA kodierende Sequenz trxA:
Zur Amplifikation mittels PCR wurden folgende Ansätze gewählt:
- Die Vektor-DNS (trxA-Expressionvektor) wurde mittels Inverse PCR mit den Primern vtrxA5-fwrd (SEQ ID Nr. 29) und 3477-rev (SEQ ID Nr. 13) amplifiziert, wobei das folgende PCR-Programm gewählt wurde: 2 min 98 °C, dann 30 Zyklen mit 45 s 98 °C / 30 s 60 °C / 2,5 min 72 °C.
- trxB5 wurde mittels PCR mit den Primern ftrxB-fwrd (SEQ ID Nr. 19) und ftrxB5-rev (SEQ ID Nr. 30) amplifiziert, wobei das folgende PCR-Programm gewählt wurde: 2 min 98 °C -> dann 30 Zyklen mit 45 s 98 °C / 30 s 60 °C / 15 s 72 °C. Als Template wurde genomische DNS von BL21 verwendet.

Nach Abschluss der PCR-Reaktionen wurde dem jeweiligen Reaktionsmix das Restriktionsenzym DpnI (10 Units, NEB) zugesetzt und der Ansatz für 1 Stunde bei 37 °C inkubiert. Im Anschluss erfolgte die chromatographische Aufreinigung der Insert-DNS (Macherey & Nagel; NucleoSpin^{®} Gel and PCR Clean-up-Kit).

Für die LIC-PCR-Reaktion wurden 50 ng der amplifizierten Vektor-DNS des trxA-Expressionsvektors und 75 ng der Insert-DNS (trxB5) verwendet.

Der LIC-PCR-Ansatz wurde dann mit Standardmethoden in *E.coli* XL1-blue-Zellen transformiert und auf selektivem LB-Medium ausplattiert (LB + 100 mg/L Ampicillin) und für 18 h bei 37 °C inkubiert. Zur Identifikation korrekter Klone wurde Plasmid-DNS aus den erhaltenen Kolonien isoliert und die Expressionskassette vollständig sequenziert.

SEQ ID Nr. 28 gibt die aus der Expression der Sequenz SEQ ID Nr. 27 resultierenden Aminosäuresequenz des Fusionsproteins TrxB5A an.

### Beispiel 4: Enzymaktivität der Cystinreduktasen

Für die rekombinante Expression der Proteine TrxA, TrxB, TrxBA, TrxB5A, TrxAB und Ml-TrxBA wurden die überprüften, das entsprechende Protein kodierenden Expressionsplasmide in den Stamm *E.coli* TOP10 (Thermo_Fisher Scientific, MA/USA) eingebracht. Die Bakterienzellen wurden auf LB-Medium mit 100 mg/L Ampicillin ausplattiert. Anschließend wurden 25 ml LB-Medium, das 100 mg/L Ampicillin und 0,2 % Arabinose (w/v) enthielt, mit einer Einzelkolonie angeimpft und 18 h bei 28°C im Kulturschüttelschrank inkubiert.

Zur Isolierung der exprimierten Proteine wurden die Zellen durch Zentrifugation sedimentiert (10 min 4000 g), der Medien-überstand entfernt und die Biomasse in Lysispuffer (PBS + 10% BugBuster (Sigma)) resuspendiert. Die Zellen wurden entsprechend der Angaben des Herstellers im Handbuch des BugBuster-Kits (Sigma) in 15 min bei RT vollständig lysiert und anschließend unlösliche Bestandteile per Zentrifugation (20 min 9500 rpm entsprechend den Herstellerangaben) abgetrennt. Zur Aufreinigung des Überstands (= Zelllysat) mittels Affinitätschromatographie wurde das erhaltene Zelllysat auf PBS-äquilibrierte Protino IDA2000-Säulen (Macherey & Nagel) aufgetragen. Nach einem Waschschritt (7 ml PBS) wurden die Proben mit 5 ml Elutionspuffer (PBS + 200 mM Immidazol) eluiert. Die erhaltenen Proteinkonzentrationen wurden mittels Bradfordassay (Thermo_Fiseher) bestimmt.

Zur Bestimmung der Cystin-Reduktase-Aktivität der verschiedenen Enzyme TrxA, TrxB, TrxBA, TrxB5A, TrxAB oder Ml-TrxBA wurden die zwei im Folgenden detailliert beschriebenen Analysemethoden verwendet.
1. Die erste Methode bestand in der photometrischen Nachweismethode, die auf dem Verbrauch von NADPH bzw. dem dadurch bedingten Abfall der Extinktionswerte bei 340 nm bei der Reduktionsreaktion basiert. Wie in Fig. 2 schematisch dargestellt, geht die enzymatische Umsetzung von Cystin bei der Reduktion zu Cystein mit dem Verbrauch des Kofaktors NADPH einher.

Für die photometrische Bestimmung wurde folgender Assayansatz verwendet, wobei das Endvolumen der Proben 1 ml betrug:
- 100 mM Phosphatpuffer pH 7,4
- 2 mM EDTA
- 0,2 mM NADPH (Sigma)
- 1 mM Cystin (Wacker Chemie AG)
- Nach Mischen aller Assaykomponenten außer dem Enzym erfolgte der Reaktionsstart durch Enzymzugabe: 10 ug Enzym TrxA, TrxB, eine Mischung aus TrxA und TrxB, TrxBA, TrxB5A, TrxAB oder Ml-TrxBA. Die Assayansätze wurden bis zur unten beschriebenen Messung bei Raumtemperatur (ca. 25 °C) inkubiert.

Die NADPH-Konzentration der verschiedenen 1 ml-Assayansätze wurde zu unterschiedlichen Zeitpunkten mit Hilfe eines Spektrophotometers (Evolution 201 UV-Vis Spektrophotometer, Thermo Fiseher Scientific) in Form der Extinktionswerte bei 340 nm gemessen. Aus den Extinktionswerten bei 340 nm wurde mittels der Software Thermo INSIGHT des Herstellers Thermo Fiseher Scientific die Enzymaktivität der verschiedenen Proben berechnet. Als Negativkontrollen wurden jeweils parallel Proben analysiert, die entweder hitzeinaktiviertes Enzym, kein Enzym, kein Cystin als Substrat oder kein NADPH als Kofaktor enthielten.

Die Enzymaktivität wurde aus der linearen Steigung am Anfang der aufgezeichneten Kurve bestimmt, wobei die Extinktions-änderung (Abnahme) über die Zeit, die Geschwindigkeit des NADPH Verbrauchs angibt. Da immer gleich viel Enzym eingesetzt wird, lassen sich die Geschwindigkeiten vergleichen.

2. Die zweite Methode wies das durch die Cystin-Reduktase-Aktivität aus Cystin entstandene Cystein unmittelbar anhand der freien SH-Gruppe durch die Reaktion mit der Verbindung DTNB (5,5'-Dithiobis-2-nitrobenzoesäure; Ellman's Reagenz) nach, wie in Fig. 3 schematisch dargestellt, im Rahmen dieser Erfindung auch mit DTNB-Assay bezeichnet.

Für die Photometrie wurde folgender Assayansatz verwendet, wobei das Endvolumen der Proben 1 ml betrugt:
- 100 mM Phosphatpuffer pH 7,4
- 2 mM EDTA
- 0,2 mM NADPH (Sigma)
- 1 mM Cystin (Wacker Chemie AG)
- Nach Mischung aller Assaykomponenten außer dem Enzym erfolgte der Reaktionsstart durch Enzymzugabe: 10 µg Enzym TrxA, TrxB, eine Mischung aus TrxA und TrxB, TrxBA, TrxB5A, TrxAB oder Ml-TrxBA. Die Assayansätze wurden bis zur unten beschriebenen Messung bei Raumtemperatur (ca. 25 °C) inkubiert.

Die Quantifizierung von L-Cystein erfolgte durch den von Lee S.-H. et al. 1995 (Biochemical and Biophysical Research Communications 213, S. 837-844) beschriebenen Test mittels 5,5'-Dithiobis-2-nitrobenzoesäure (DTNB). Die Quantifizierung von Cys-TNB erfolgte durch Messung der DTNBvermittelten Extinktion bei 412 nm. Eine Analyse ist auch über HPLC möglich.

Als Negativkontrollen wurden jeweils parallel Proben analysiert, die entweder hitzeinaktiviertes Enzym, kein Enzym, kein Cystin als Substrat oder kein NADPH als Kofaktor enthielten.

Fig. 4 zeigt die relative Cystin-Reduktase-Aktivität der Enzyme TrxBA, TrxB5A, TrxAB und Ml-Trx sowie einer (9:1)-Mischung von TrxA und TrxB nach photometrischer Bestimmung wie oben unter Punkt 1 beschrieben, wobei die Aktivität der (9:1)-Mischung von TrxA und TrxB auf 1 normiert und alle anderen Aktivitäten in Relation dazu gesetzt wurden. Bei der (9:1)-Mischung von TrxA und TrxB handelt es sich um ein abgeschätztes Mischungsverhältnis der Einzelenzyme, dass sich in Vergleichsversuchen als aktivstes Mischungsverhältnis herausgestellt hat.

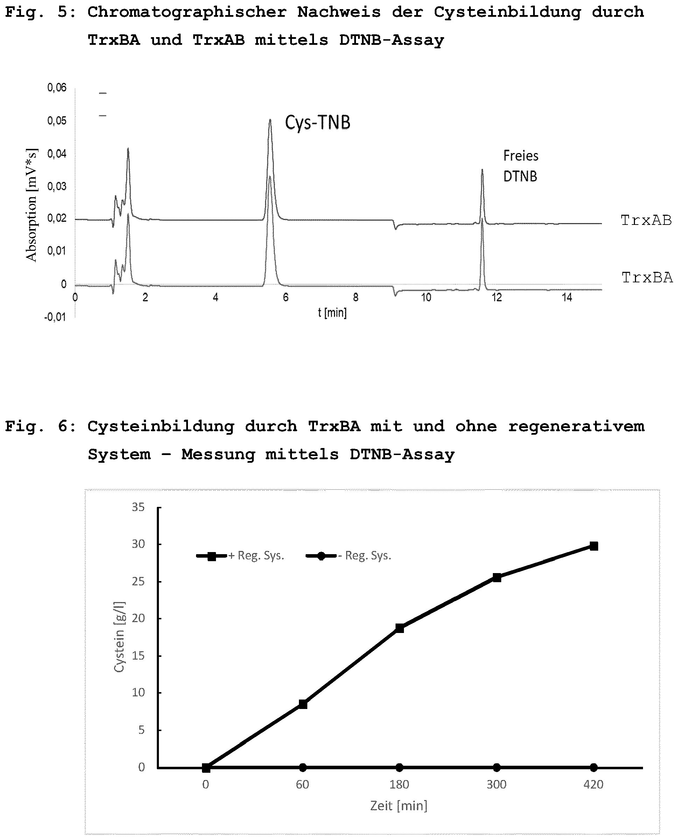

Fig. 5 zeigt den Nachweis der Cysteinbildung durch TrxBA und TrxAB nach DTNB-Assay und HPLC wie oben unter Punkt 2 beschrieben.

Der Einsatz von Klonen, die eine längere Linkersequenz als TrxB5A im Bereich von ≥60 Nukleotiden zwischen den für TrxA und TrxB cds enthielten, führten erstaunlicher Weise zu einer signifikant geringeren Aktivität des Fusionsproteins.

### Beispiel 5: Aktivität der Enzymkombination Cystinreduktase und ADH

Vergleicht man die Enzymspezifität gegenüber den Kofaktoren NADPH und NADP⁺, so sind die Cystinreduktasen TrxBA und TrxAB hochspezifisch für den Kofaktor NADPH. In den Tests konnte mit NADP⁺ keine Enzymaktivität gegenüber Cystin nachgewiesen werden.

In Kombinationsexperimenten wurde untersucht, in wieweit das Enzym Alkoholdehydrogenase (ADH) NADP⁺ für die Reaktion wieder in NADPH überführen kann.

Hierzu wurden folgende Bedingungen verwendet:
- 100 mM Phosphatpuffer pH 7.4
- 5 µl Isopropanol
- 2 mM EDTA
- 15-50 µM NADPH oder NADP⁺
- 1 mM Cystin
- 5 µg Enzym TrxBA
- 50 µl Zelllysat-Rohextrakt oder als negativ Kontrolle 50 µl Phosphat-Puffer (Fast Prep Aufschluss von 0,5 ml Zellen in 1,5 ml 4x Phosphat-Puffer pH 7,4. Herstellung der Zellen wie in EP 1 832 658 B1 beschrieben)

Die Reaktion wurde für 60 min bei 30°C inkubiert. In 5 min. Abständen wurden Proben entnommen und das freigesetzte Cystein mit DTNB wie in WO 2013/000864 A1 beschrieben derivatisiert. Die Quantifizierung erfolgte durch Messung der DTNBvermittelten Extinktion bei 412 nm.

Fig. 6 zeigt das Ergebnis der Messung der Cysteinbildung durch TrxBA mittels DTNB-Assay in Gegenwart (mit) oder in Abwesenheit (ohne) ADH als regenerativem System.

### In den Figuren verwendete Abkürzungen

AraC: AraC Gen (Repressorgen)
pAraC: Promotor des AraC Gens (Repressorgen; rev-Orientierung zum P_{BAD})
pBAD (im Rahmen dieser Erfindung auch mit P_{BAD} bezeichnet):
   Arabinose-induzierbarer Promotor zur Expression (downstream) eingefügter Zielprotein-Sequenzen
6HIS: kodierende Region für His-tag
term: Terminator für die Transkription
Amp: Ampicillin-Resistenzmarker
CR: Cystinreduktase
rel.: relativ
bps: Basenpaare
t: Zeit

## Patentansprüche

1. Enzym zur Reduktion von Cystin zu Cystein, **dadurch gekennzeichnet, dass** es sich beim Enzym um ein Fusionsprotein handelt, das die Proteinaktivitäten von
i) Thioredoxin (Protein i) mit der KEGG-Datenbank Nummer EC 1.8.4.8 oder EC 1.8.4.10,
wobei es sich bei Thioredoxin (Protein i) um die Proteinaktivität von Thioredoxin 1 aus *E.coli* handelt, und
ii) Thioredoxin-Reduktase (Protein ii) mit der KEGG-Datenbank Nummer EC 1.8.1.9 umfasst,
wobei es sich bei der Thioredoxin-Reduktase (Protein ii) um die Proteinaktivität der Thioredoxin-Reduktase aus *E.coli* handelt,
wobei die Aktivität des Fusionsproteins mindestens 100% der Aktivität einer Mischung der gleichen, aber nicht fusionierten Einzelproteine i und ii beträgt,
wobei das Fusionsprotein die Enzymaktivität aufweist, Cystin zu Cystein zu reduzieren und
wobei die für die Aktivität von Protein i und ii verantwortlichen kodierenden Sequenzen (cds) fusioniert wurden.

2. Enzym gemäß Anspruch 1, **dadurch gekennzeichnet, dass** das Fusionsprotein zwei Aminosäure-Sequenzen umfasst, wobei
- die eine Aminosäure-Sequenz eine Identität von mindestens 50% zu SEQ ID Nr. 7 hat und
- die andere Aminosäure-Sequenz eine Identität von mindestens 50% zu SEQ ID Nr. 8 hat und
wobei das Fusionsprotein CR-Aktivität (Cystinreduktase-Aktivität) besitzt.

3. Enzym gemäß einem oder mehreren der Ansprüche 1 oder 2, **dadurch gekennzeichnet, dass** im Fusionsprotein die Aminosäuresequenz des N-terminal gelegenen Proteins C-terminal um eine bis maximal fünf Aminosäuren verkürzt ist.

4. Enzym gemäß einem oder mehreren der Ansprüche 1 bis 3,
**dadurch gekennzeichnet, dass** im Fusionsprotein die Aminosäuresequenz des C-terminal gelegenen Proteins N-terminal um ein bis maximal fünf Aminosäuren verkürzt ist.

5. Enzym gemäß einem oder mehreren der Ansprüche 1 bis 4,
**dadurch gekennzeichnet, dass** im Fusionsprotein die Aminosäuresequenz der fusionierten Proteinaktivitäten von Thioredoxin (Protein i) und Thioredoxin-Reduktase (Protein ii) durch eine Linkersequenz von einer bis höchstens fünf Aminosäuren verbunden ist.

6. Enzym gemäß einem oder mehreren der Ansprüche 1 bis 5,
**dadurch gekennzeichnet, dass** es sich beim Fusionsprotein um eine der Aminosäure-Sequenzen ausgewählt aus der Gruppe bestehend aus SEQ ID Nr. 9, SEQ ID Nr. 10 und SEQ ID Nr. 28 handelt.

7. Verfahren zur enzymatischen Reduktion von Cystin zu Cystein, **dadurch gekennzeichnet, dass** Cystin mit einem Enzym gemäß einem der Ansprüche 1 bis 6 in Gegenwart eines Kofaktors reduziert wird,
wobei aus einem Molekül der chemischen Verbindung Cystin zwei Moleküle L-Cystein entstehen.

8. Verfahren gemäß Anspruch 7, **dadurch gekennzeichnet, dass** die Reduktion bei einem pH-Wert von 6 bis 9 erfolgt.

9. Verfahren gemäß einem oder mehreren der Ansprüche 7 oder 8, **dadurch gekennzeichnet, dass** es sich beim Kofaktor um eine Substanz ausgewählt aus der Gruppe bestehend aus NADPH und NADH handelt.

10. Verfahren gemäß einem oder mehreren der Ansprüche 7 bis 9, **dadurch gekennzeichnet, dass** es ein Kofaktor-regenerierendes Enzym enthält.

11. Verfahren gemäß Anspruch 10, **dadurch gekennzeichnet, dass** es sich bei dem Kofaktor-regenerierenden Enzym um eine Dehydrogenase handelt, wobei die Reduktion zusätzlich in Gegenwart eines Elektronendonors erfolgt.

12. Verfahren gemäß Anspruch 11, **dadurch gekennzeichnet, dass** es sich bei der Dehydrogenase um die Alkoholdehydrogenase handelt, wobei als Elektronendonor Isopropanol verwendet wird.

## Claims

1. Enzyme for reducing cystine to cysteine, **characterized in that** the enzyme is a fusion protein that comprises the protein activities of
i) thioredoxin (protein i) having KEGG database number EC 1.8.4.8 or EC 1.8.4.10,
wherein thioredoxin (protein i) is the protein activity of thioredoxin 1 from *E. coli,* and
ii) thioredoxin reductase (protein ii) having KEGG database number EC 1.8.1.9,
wherein the thioredoxin reductase (protein ii) is the protein activity of the thioredoxin reductase from *E. coli,*
wherein the activity of the fusion protein is at least 100% of the activity of a mixture of the same but unfused individual proteins i and ii,
wherein the fusion protein has the enzyme activity to reduce cystine to cysteine and
wherein the coding sequences (cds) responsible for the activity of protein i and ii have been fused.

2. Enzyme according to Claim 1, **characterized in that** the fusion protein comprises two amino acid sequences, wherein
- one of these amino acid sequences is at least 50% identical to SEQ ID No. 7 and
- the other amino acid sequence is at least 50% identical to SEQ ID No. 8 and
wherein the fusion protein has CR activity (cystine reductase activity).

3. Enzyme according to one or both of Claims 1 and 2, **characterized in that** the amino acid sequence of the protein located N-terminally is in the fusion protein shortened C-terminally by one to at most five amino acids.

4. Enzyme according to one or more of Claims 1 to 3, **characterized in that** the amino acid sequence of the protein located C-terminally is in the fusion protein shortened N-terminally by one to at most five amino acids.

5. Enzyme according to one or more of Claims 1 to 4, **characterized in that** the amino acid sequence of the fused protein activities of thioredoxin (protein i) and thioredoxin reductase (protein ii) are in the fusion protein connected by a linker sequence of one to at most five amino acids.

6. Enzyme according to one or more of Claims 1 to 5, **characterized in that** the fusion protein is an amino acid sequence selected from the group consisting of SEQ ID No. 9, SEQ ID No. 10, and SEQ ID No. 28.

7. Process for enzymatically reducing cystine to cysteine, **characterized in that** cystine is reduced by an enzyme according to any of Claims 1 to 6 in the presence of a cofactor,
wherein two molecules of L-cysteine are formed from one molecule of the chemical compound cystine.

8. Process according to Claim 7, **characterized in that** the reduction takes place at a pH of from 6 to 9.

9. Process according to one or both of Claims 7 and 8, **characterized in that** the cofactor is a substance selected from the group consisting of NADPH and NADH.

10. Process according to one or more of Claims 7 to 9, **characterized in that** it includes a cofactor-regenerating enzyme.

11. Process according to Claim 10, **characterized in that** the cofactor-regenerating enzyme is a dehydrogenase, with the reduction additionally taking place in the presence of an electron donor.

12. Process according to Claim 11, **characterized in that** the dehydrogenase is the alcohol dehydrogenase, with isopropanol used as the electron donor.

## Revendications

1. Enzyme pour la réduction de cystine en cystéine, **caractérisée en ce qu'**il s'agit, pour l'enzyme, d'une protéine de fusion qui comprend les activités protéiniques de
i) la thiorédoxine (protéine i) présentant le numéro de la base de données KEGG EC 1.8.4.8 ou EC 1.8.4.10, où il s'agit, pour la thiorédoxine (protéine i), de l'activité protéinique de la thiorédoxine 1 d'E. coli et
ii) la thiorédoxine-réductase (protéine ii) présentant le numéro de la base de données KEGG EC 1.8.1.9, où il s'agit, pour la thiorédoxine-réductase (protéine ii), de l'activité protéinique de la thiorédoxine-réductase d'E. coli,
l'activité de la protéine de fusion représentant au moins 100% de l'activité d'un mélange des mêmes protéines individuelles i et ii, mais non fusionnées, la protéine de fusion présentant l'activité enzymatique de réduction de la cystine en cystéine et les séquences codantes, responsables de l'activité des protéines i et ii (cds) ayant été fusionnées.

2. Enzyme selon la revendication 1, **caractérisée en ce que** la protéine de fusion comprend deux séquences d'acides aminés, où
- l'une séquence d'acides aminés présente une identité d'au moins 50% avec la séquence SEQ ID NO. 7 et
- l'autre séquence d'acides aminés présente une identité d'au moins 50% avec la séquence SEQ ID NO. 8 et la protéine de fusion présentant une activité de CR (activité de cystine-réductase).

3. Enzyme selon l'une ou plusieurs des revendications 1 à 2, **caractérisée en ce que**, dans la protéine de fusion, la séquence d'acides aminés de la protéine située en position N-terminale est raccourcie, du côté C-terminal, d'un à au maximum cinq acides aminés.

4. Enzyme selon l'une ou plusieurs des revendications 1 à 3, **caractérisée en ce que**, dans la protéine de fusion, la séquence d'acides aminés de la protéine située en position C-terminale est raccourcie, du côté N-terminal, d'un à au maximum cinq acides aminés.

5. Enzyme selon l'une ou plusieurs des revendications 1 à 4, **caractérisée en ce que**, dans la protéine de fusion, la séquence d'acides aminés des activités protéiniques fusionnées de thiorédoxine (protéine i) et de thiorédoxine-réductase (protéine ii) est liée par une séquence de lieur d'un à au plus cinq acides aminés.

6. Enzyme selon l'une ou plusieurs des revendications 1 à 5, **caractérisée en ce qu'**il s'agit, pour la protéine de fusion, d'une des séquences d'acides aminés choisies dans le groupe constitué par les séquences SEQ ID NO. 9, SEQ ID NO. 10 et SEQ ID NO. 28.

7. Procédé pour la réduction enzymatique de cystine en cystéine, **caractérisé en ce que** la cystine est réduite par une enzyme selon l'une quelconque des revendications 1 à 6 en présence d'un cofacteur, deux molécules de L-cystéine étant formées à partir d'une molécule du composé chimique cystine.

8. Procédé selon la revendication 7, **caractérisé en ce que** la réduction est réalisée à un pH de 6 à 9.

9. Procédé selon l'une ou plusieurs des revendications 7 à 8, **caractérisé en ce qu'**il s'agit, pour le cofacteur, d'une substance choisie dans le groupe constitué par NADPH et NADH.

10. Procédé selon l'une ou plusieurs des revendications 7 à 9, **caractérisé en ce qu'**il contient une enzyme régénérant le cofacteur.

11. Procédé selon la revendication 10, **caractérisé en ce qu'**il s'agit, pour l'enzyme régénérant le cofacteur, d'une déshydrogénase, la réduction étant en outre réalisée en présence d'un donneur d'électrons.

12. Procédé selon la revendication 11, **caractérisé en ce qu'**il s'agit, pour la déshydrogénase, de l'alcool-déshydrogénase, de l'isopropanol étant utilisé comme donneur d'électrons.
